# FASCICULE DE BREVET EUROPEEN

(11) **EP 4 293 470 B1**
(45) Date de publication et mention de la délivrance du brevet: **06.08.2025**
(21) Numéro de dépôt: 23177289.8
(22) Date de dépôt: 05.06.2023
(51) Int. Cl.: G05D 11/13

(54) **MÉTHODE DYNAMIQUE POUR REMPLIR RAPIDEMENT DES LOTS IDENTIQUES DE BOUTEILLES DE MÉLANGES GAZEUX À HAUTE PRÉCISION MÉTROLOGIQUE**
DYNAMISCHES VERFAHREN ZUM SCHNELLEN FÜLLEN VON IDENTISCHEN FLASCHENCHARGEN AUS GASGEMISCHEN MIT HOHER MESSGENAUIGKEIT
DYNAMIC METHOD FOR RAPIDLY FILLING IDENTICAL BATCHES OF BOTTLES WITH HIGH METROLOGICAL PRECISION GAS MIXTURES

(30) Priorité: 17.06.2022 FR 2205967
(43) Date de publication de la demande: 20.12.2023
(73) Titulaire: L'AIR LIQUIDE, SOCIETE ANONYME POUR L'ETUDE ET L'EXPLOITATION DES PROCEDES GEORGES CLAUDE, 75007 Paris (FR)
(72) Inventeur: LE GENDRE, Erwann, 38360 Sassenage (FR); DAVIET, Christian, 78350 Les Loges-En-Josas (FR); WETERINGS, Werner, 4817 BL Breda (NL); KOCH, Maurice, 40235 Duesseldorf (DE)
(74) Mandataire: Air Liquide

(56) Documents cités:
- EP-B1- 0 877 196
- EP-B1- 2 153 109
- FR-A1- 2 532 858
- FR-A1- 2 811 909

## Description

La présente invention concerne le domaine de l'analyse des gaz et s'intéresse tout particulièrement aux domaines de la fabrication et du conditionnement des mélanges gazeux pour les procédés de calibration.

Les bouteilles de calibration gaz sont utilisées dans de nombreuses applications. La calibration des analyseurs gaz qui contrôlent les émissions gazeuses des véhicules légers et lourds motorisés est un exemple d'application.

Les bouteilles de calibration de mélange gazeux doivent respecter les normes internationales en vigueur. Ces normes exigent une incertitude bien définie sur la concentration des constituants du mélange gazeux (voir par exemple les normes ISO 17025 et ISO 17034).

Cette incertitude sur la concentration des constituants d'un mélange gazeux représente un challenge lors de telles fabrications de mélanges, entraînant notamment un contrôle strict des conditions de remplissage.

Il existe différents types de méthodes de remplissage, par exemple des méthodes gravimétriques, ou encore des méthodes dynamiques. Lors d'un remplissage par une méthode gravimétrique, les bouteilles sont remplies avec une méthode réglée initialement, qui n'est pas modifiée par la suite, ou bien qui peut être modifiée de manière déterministe pendant la production des bouteilles.

Le document EP-877 196 illustre le domaine de la préparation de mélanges gazeux de précision.

Comme on le verra plus en détails dans ce qui suit, la présente invention propose une nouvelle méthode de remplissage dynamique qui permet la production de lots identiques de bouteilles, elles-mêmes identiques, de mélanges gazeux de référence, ceci de manière rapide, flexible et asservie sur la composition cible, avec un très grand niveau d'exactitude au sens métrologique du terme.

Le mélange gazeux peut être composé d'un constituant dans une matrice (mélange binaire) ou bien de plusieurs constituants dans une matrice (mélange multi-composants), la matrice pouvant elle-même être composée de plusieurs constituants, par exemple un air synthétique.

La présente invention a notamment pour objectifs de :
- produire en une seule fois un lot de bouteilles identiques, allant même jusqu'à des nombres de bouteilles importants, par exemple jusqu'à 24 bouteilles.
- produire des séries de lots identiques de bouteilles complètement identiques.
- produire un nouveau lot de bouteilles identique à un lot produit lors d'une opération précédente ou d'une période précédente.
- livrer un certificat de conformité identique valable pour toutes les bouteilles d'un lot ou même pour plusieurs lots de bouteilles.
- produire un lot de bouteilles à partir d'une bouteille dont la concentration est connue ou bien inconnue (sous réserve de pouvoir l'analyser en préalable au remplissage).
- produire un nouveau de lot de bouteilles en remplacement à l'identique d'un lot défaillant ou pollué durant l'utilisation.

Ces objectifs une fois atteints permettent à un site utilisateur de commander une bouteille ou un lot de bouteilles ou bien une série de lots de bouteilles identiques à celle(s) ou ceux déjà utilisé(e)s sans avoir à modifier sa méthode de calibration interne.

La présente invention permet ainsi également de produire (voire même de reproduire) une bouteille ou un lot de bouteilles ou bien une série de lots de bouteilles, à partir d'une bouteille qui sert de référence interne pour le site utilisateur, cette bouteille référence pouvant être de toute origine sous réserve de pouvoir l'analyser en préalable au remplissage demandé.

Ces points aident les utilisateurs à gagner en efficacité et productivité en limitant voire en supprimant les variabilités et le temps passé sur le réglage et l'ajustement de leurs méthodes d'analyse. Notamment en n'ayant pas à changer la valeur de concentration du gaz de calibration dans la méthode analytique lors d'un changement de bouteille de calibration.

La présente invention s'attache également à permettre, notamment, de :
- produire un lot de bouteilles avec un débit moyen (par exemple 50 m³/heure) ou avec un fort débit (par exemple 100 m³/heure) pour réduire le temps de remplissage.
- produire un lot de 24 bouteilles identiques en environ 2 heures avec un fort débit.
- produire un lot de 12 bouteilles identiques en environ 2 heures avec un débit moyen.
- produire un lot de 3 bouteilles identiques en environ 30 minutes avec un débit moyen.
- Enchaîner très rapidement (typiquement moins de 30 minutes) la production d'une bouteille ou d'un lot de bouteilles d'une nouvelle composition à la suite de la production de lot de bouteilles d'une première composition différente.

Ces points permettent d'accélérer le processus de production, augmentant le taux de disponibilité de l'installation de remplissage, améliorant sa productivité pour atteindre des productivités élevées, jusqu'à 4 à 6 lots de 24 bouteilles par jour, réduisant ainsi le délai entre la commande et la livraison du produit au site utilisateur.

La présente invention s'attache également à permettre de :
- analyser simultanément, en ligne et avec une grande exactitude l'ensemble des constituants lors du remplissage.
- calculer précisément la concentration obtenue de chaque composant à l'issue du remplissage.
- calculer précisément la concentration atteinte tout au long du remplissage et ainsi suivre le bon déroulement du remplissage.
- contrôler automatiquement la fabrication du lot de bouteilles dynamiquement à l'aide d'un cycle de rétroaction.
- automatiser et contrôler de manière dynamique et asservie à la composition cible la production du lot de bouteilles de mélange gazeux.
- produire un lot de bouteilles en garantissant la concentration obtenue à partir de la concentration calculée en temps réel et sans avoir nécessairement à analyser le lot de bouteilles après le remplissage.

Ces points permettent d'arrêter au plus tôt le processus de remplissage en cas d'anomalie, ou bien décider que le produit sera conforme ou au moins acceptable à la fin du remplissage.

La présente invention s'attache également à permettre de :
- produire le mélange sans contaminants et impuretés externes.
- produire une bouteille ou un lot de bouteilles avec une erreur de réalisation inférieure à 0.5% relatif sur chaque composant du mélange cible.
- produire une bouteille ou un lot de bouteilles avec une incertitude relative élargie (k=2) inférieure à 1% sur chaque composant.
- produire un lot de bouteilles homogène entre elles avec un facteur « S_{bb} » inférieur à 0.1% (« S_{bb} » facteur internationalement utilisé pour la notion de « Between Bottle Homogeneity »).
- produire une série de lots de bouteilles avec une homogénéité inférieure à 0.71% relatif sur chaque composant du mélange (des lots de bouteilles identiques).
- produire des mélanges conformes aux exigences des normes ISO 17025 et ISO 17034.

Ces points permettent de produire des mélanges gaz avec une grande traçabilité et connaissance des incertitudes associées. Ces informations sont nécessaires pour certains sites utilisateurs et certains domaines d'application. Et on sait notamment que dans le domaine de la mesure des émissions automobiles la traçabilité et l'incertitude des mélanges de référence sont strictement contrôlées et réglementées.

La présente invention s'attache également à permettre de :
- Passer facilement de la production d'une gamme de concentration à un autre (allant du ppm.mol au %.mol) ainsi que de certaines molécules d'intérêt à d'autres (CO et/ou CO₂ et/ou O₂ et/ou NO et/ou CO₂ et/ou CH₄ et/ou C₃H₈ etc... dans une matrice variée, N₂ ou Air Synthétique ou autre...).

Ce point permet une grande flexibilité et une grande productivité dans l'utilisation du mélangeur dynamique en s'adaptant rapidement à la demande des sites utilisateurs et aux besoins de nouveaux marchés qui naîtraient.

La présente invention s'attache également à permettre de :
- analyser simultanément l'ensemble des constituants du mélange en post-remplissage
- réaliser semi-automatiquement une analyse post-remplissage d'une seule bouteille ou de chaque bouteille dans la foulée de la production d'un lot de bouteilles.

Cela permet également d'analyser en fin de production une bouteille ou bien, l'une après l'autre, chaque bouteille de tout ou partie d'un lot de bouteilles afin de :
i. contrôler la concentration exacte du mélange gazeux dans la bouteille ou dans chaque bouteille de tout ou partie d'un lot.
ii. contrôler l'écart de réalisation entre la concentration finale et la concentration cible pour la ou chaque bouteille de tout ou partie d'un lot.
iii. contrôler l'écart d'homogénéité entre chaque bouteille de tout ou partie d'un même lot (voire entre plusieurs lots de bouteilles produits).
iv. réaliser l'ensemble des analyses demandées par un site utilisateur.
v. réaliser l'ensemble des analyses demandées par les normes internationales.

On note par ailleurs que l'invention permet de réaliser l'analyse au même endroit que le remplissage, économisant ainsi le temps de déplacement d'une bouteille ou d'un lot de bouteilles, évitant les risques de manipulation associées au déplacement de bouteilles, les risques de contaminations externes en débranchant et rebranchant les connectiques gaz, et les risques de non disponibilité ou d'engorgement des autres moyens d'analyse du site.

La présente invention s'attache également à permettre de :
- produire un lot de bouteilles avec un mélange gazeux à la même pression cible.
- produire un lot de bouteilles en arrêtant automatiquement le remplissage à la pression cible, étant pris en compte le fait que la dynamique de remplissage provoque un échauffement du mélange au niveau de la bouteille, la pression durant le remplissage s'en trouvant augmentée en conséquence.

Ces points assurent une qualité et une fiabilité optimale lors de la fabrication d'une bouteille ou d'un lot de bouteille en offrant la possibilité au site utilisateur de choisir la pression souhaitée et d'obtenir systématiquement la même quantité de matière dans la bouteille et/ou dans chaque bouteille du lot.

La présente invention s'attache également à permettre de :
- produire un lot de bouteilles en tolérant des aléas de production tels que des baisses de pression et/ou des courts arrêts d'alimentation (sous réserve de non pollution et que les aléas n'interviennent pas trop tard en fin de cycle de remplissage).
- produire un lot de bouteilles en minimisant la quantité de gaz perdu notamment lors du réglage des débits cibles.
- éviter les reprises de production défaillant ou non conforme par exemple si il y a un écart de réalisation trop important.
- produire un lot de bouteilles sans avoir à remplir des bouteilles factices, en offrant un principe de fonctionnement ne nécessitant pas un nombre minimal de bouteilles (les performances de remplissage étant garanties de 2, 3 bouteilles à 24 voire plus).

Ces points permettent une haute fiabilité du système de mélange en s'affranchissant des erreurs aléatoires inhérentes à tout système électronique.

Le système de mélange s'adapte automatiquement pendant le remplissage d'une bouteille ou d'un lot de bouteille pour rectifier ces erreurs afin de s'approcher au plus près de la concentration cible. Cette haute fiabilité permet d'optimiser les cycles de production en réduisant drastiquement les erreurs de production.

Examinons maintenant dans ce qui suit les solutions disponibles dans l'art antérieur de ce domaine et leurs limitations.

On peut résumer ces solutions de la façon suivante :
- Limitation 1 : Les systèmes de mélange existant comportent de nombreuses étapes à déclencher manuellement et conditionnellement à l'initiative et au jugement de l'opérateur qualifié, sous l'éclairage quasi indispensable d'analyses intermédiaires et finales. Ces tâches limitent la capacité de production, la rapidité de remplissage et peuvent également être des sources d'erreurs comme la variabilité inter-opérateurs augmentant l'incertitude sur les mélanges produits.
- Limitation 2 : La différence de concentration et d'incertitude entre différents lots de bouteilles d'un même mélange est souvent significative. Cette limitation conduit régulièrement à ce que le site soit livré avec un nouveau lot de concentration différente des précédents et/ou avec un écart de réalisation plus ou moins important par rapport à son attente. Ce qui oblige les sites utilisateurs à réaliser l'ensemble de leur procédure de calibration à chaque nouveau lot de bouteilles.
- Limitation 3 : Si la différence de concentration et d'incertitude est trop importante, il est certes possible de proposer au site utilisateur le lot hors spécification sous dérogation, ce qui peut prendre un temps non négligeable de discussion, de négociation et d'immobilisation des moyens et emballages.
- Limitation 4 : Afin de limiter la différence de concentration et d'incertitude entre différents lots de bouteilles d'un même mélange, il est souvent nécessaire d'ajouter des étapes manuelles de compléments de remplissage pour atténuer la différence constatée. Il peut même parfois être nécessaire de procéder à une purge complète du lot pour relancer son remplissage depuis le début, sauf à traiter le lot sous dérogation (comme mentionné en limitation 3).
- Limitation 5 : Afin d'évaluer la différence de concentration et d'incertitude entre différents lots de bouteille d'un même mélange et ainsi évaluer le besoin d'ajouter des étapes manuelles de compléments de remplissage pour atténuer la différence constatée, il y a lieu de réaliser une ou des phases d'analyse intermédiaires et/ou finales en laboratoire qui nécessitent le déplacement des bouteilles ainsi qu'un temps logistique significatif.
- Limitation 6 : Les solutions existantes manquent de flexibilité. Une fois que la solution est installée, il est très difficile de modifier la gamme des constituants ou la gamme de concentrations du cadre initial.
- Limitation 7 : Les solutions existantes n'intègrent pas de système d'asservissement et de cycle de rétroaction. Le cycle de remplissage n'est pas géré automatiquement et dynamiquement limitant drastiquement le contrôle et la vérification en temps réel de la concentration finale. La méthode initialement réglée n'est pas modifiée ou bien est modifiée de manière déterministe et manuelle pendant la production du lot de bouteilles.
- Limitation 8 : Comme les solutions existantes n'intègrent pas de système d'asservissement et de cycle de rétroaction, la production de bouteilles ou de lots de bouteilles subit les erreurs aléatoires des systèmes de mesure et de débit lors du remplissage. Il est ainsi très difficile de produire des lots de bouteilles parfaitement identiques (typiquement avec une homogénéité inter-lot inférieure à 0.71% relatif).

Comme on va le voir ci-après, la solution de la présente invention lève l'ensemble de ces limitations.

Décrivons, dans ce qui suit, la présente invention.

Les gaz de fond, appelés aussi gaz « matrice » (généralement Azote ou Air Synthétique) sont stockés dans un réservoir.

Les gaz purs (tels CO et/ou CO2 et/ou C3H8 et/ou CH4 et/ou NO et/ou O₂ etc...) qui vont être utilisés pour réaliser les mélanges gazeux, appelés aussi « gaz de mélange» sont stockés dans des bouteilles à haute pression.

Les gaz de références, utilisés pour étalonner le système analytique, sont stockés dans des bouteilles à haute pression.

Les lignes de gaz qui acheminent le gaz de fond et le gaz de mélange pour un mélange binaire ou les différents gaz de mélange pour un mélange multi-composants, vers des régulateurs de débit massique, sont habituellement filtrés, parfois chauffés, et régulés en pression, par exemple à 4 bars.

Des régulateurs de débit massique sont ici spécifiquement choisis et mis en œuvre pour contrôler de manière extrêmement stable et en même temps réactive, les débits partiels des gaz de mélanges et des gaz de fond. Le nombre de régulateurs massique mis en œuvre peut être facilement modifié selon les besoins.

Chaque ligne de gaz (gaz de fond et gaz de mélange) dont le débit est régulé par des régulateurs massiques, est injectée dans une chambre de mélange.

Les différents constituants (gaz de fond + gaz de mélange) sont intelligemment mélangés : suppression du risque explosif lors de l'injection combinée de O₂ (injection en amont de la chambre de mélange) et d'hydrocarbures tels que le propane (injection en aval de la chambre de mélange) ; homogénéisation évitant la formation de veine de gaz via l'injection des gaz dans la chambre de mélange à géométrie particulière, créant ainsi en toute sécurité un mélange gazeux homogène.

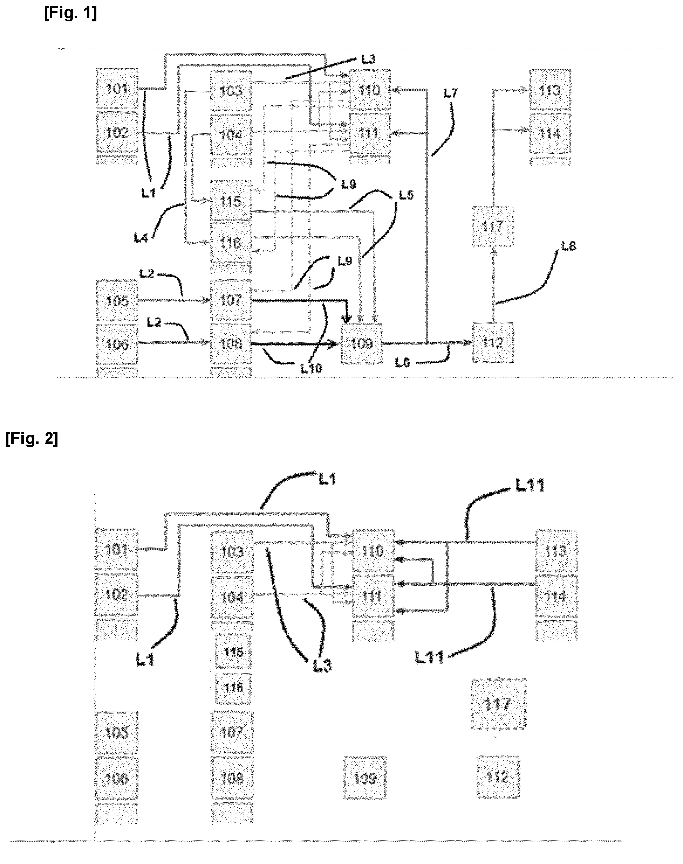

L'invention proposée ici a ceci de remarquable qu'elle intègre un système analytique capable d'analyser simultanément en temps réel l'ensemble des constituants du mélange gazeux en sortie de la chambre de mélange et avant son entrée dans l'étage de compression (Ligne L7 sur la Figure 1 annexée) voire éventuellement en entrée de la rampe de remplissage (Ligne L11 sur la Figure 2 annexée).

Une connexion gazeuse est ainsi établie entre la sortie de la chambre de mélange et le système analytique afin d'analyser en dérivation et en temps réel, la composition du mélange gazeux.

Le mélange gazeux en sortie de la chambre de mélange est dirigé vers le compresseur, compresseur qui permet d'augmenter la pression du gaz en passant de quelques bars (4-5) à la pression souhaitée.

La pression de remplissage souhaitée peut être comprise typiquement entre 50 bars et 200 bars.

Le mélange gazeux à haute pression en sortie de compresseur est alors dirigé vers une ou plusieurs rampes de remplissage permettant de répartir le mélange gazeux de manière homogène dans un lot de bouteilles (par exemple de 2 jusqu'à 24 bouteilles en parallèle).

Une fois la consigne de la pression atteinte dans une des bouteilles du lot de bouteilles, le remplissage s'arrête et la production est considérée comme terminée, et l'estimation précise de la composition du mélange dans les bouteilles ainsi remplies est immédiatement connue.

Le système analytique a été préalablement étalonné à l'aide des bouteilles de gaz de référence et d'une procédure permettant une justesse de réponse inférieure à 0.5% relatif.

Le système analytique est équipé d'analyseurs permettant d'obtenir un temps de réponse des analyseurs aussi court que possible, typiquement inférieur à la minute, et une dérive de chaque mesure le temps de remplissage aussi minime que possible, dérive typiquement inférieure à 0,25% de la mesure sur deux heures (durée qui correspond approximativement au temps de remplissage de 24 bouteilles à 200 bars de pression).

Et ainsi :
- En fonction de la composition du mélange gazeux sortant de la chambre de mélange à un instant donné ; et
- En fonction de la composition calculée et estimée en temps réel du mélange gazeux dans les bouteilles du lot par intégration depuis le début du remplissage de la composition du mélange gazeux sortant de la chambre de mélange ; et
- En fonction de la concentration finale visée ;
le système est apte, en temps réel et automatiquement, à modifier, si besoin, via une boucle de rétroaction, les débits de gaz de chaque composant du mélange, gaz(s) de mélange comme gaz(s) de fond, par modification du réglage des régulateurs de débit massique associés à chaque composant, de manière à maintenir et/ou corriger à la hausse et/ou à la baisse les concentrations du mélange sortant de la chambre de mélange, permettant ainsi de poursuivre le remplissage avec une composition nominale (situation que l'on peut qualifier de "Maintenir") ou avec une composition enrichie (situation que l'on peut qualifier de "Corriger à la hausse") ou avec une composition appauvrie (situation que l'on peut qualifier de "Corriger à la baisse") pour chaque composant du mélange.

Tout au long du remplissage, ce mécanisme d'ajustement permet de converger de manière efficace, précise et fiable vers la composition visée et ce, malgré d'éventuels aléas intervenant durant le remplissage.

Les aléas peuvent être variés, on peut citer ici celui d'une baisse de pression du réseau de gaz de fond (par exemple, d'azote) qui génère une baisse d'un débit de ce gaz de fond pendant quelques dizaines de secondes voire quelques minutes, ce qui provoque une hausse effective de la concentration de chacun des autres composants du mélange, et ainsi pendant ces quelques dizaines de secondes un enrichissement effectif, non attendu et non souhaité du mélange injecté dans chaque bouteille du lot.

On a évoqué ci-dessus la notion d' « intégration », et l'on doit comprendre ici que les situations suivantes peuvent être envisagées :
- l'intégration peut se faire en considérant que le débit de remplissage est constant tout le long du remplissage, ce qui est un premier choix de conception ;
- mais on peut envisager également une variation du débit de remplissage au cours de remplissage, et alors l'intégration peut se faire par l'intégrale pondérée par la mesure de débit en sortie de la chambre de mélange (qui est disponible au niveau du mélangeur) mais qui peut également se faire par la mesure de débit optionnel (élément 117 sur la Figure 1 annexée).

Le procédé de remplissage proposé selon la présente invention permet :
i. de détecter et de mesurer l'enrichissement effectif et donc d'en tenir compte dans le calcul d'estimation de la composition du gaz à l'intérieur des bouteilles pendant toute la durée du remplissage ;
ii. de corriger à la baisse la composition du mélange généré en sortie de la chambre de mélange pour arrêter d'enrichir la composition du gaz à l'intérieur des bouteilles voire même commencer à ré-appauvrir ce gaz pour revenir vers la composition cible souhaitée en fin de remplissage (situation « corriger à la baisse») ;
iii. une fois, l'aléa et cette correction transitoire passés (sur une durée qui peut être de quelques minutes), le mode de contrôle et régulation utilisé pourra ordonner le retour à des conditions nominales de remplissage (situation « Maintenir »).

A l'inverse, on aurait pu citer le cas d'un aléa constitué par une baisse de pression d'une alimentation bouteille d'un des composants du mélange qui provoquerait un appauvrissement de la concentration de ce composant et qui aurait à être compensé par un enrichissement transitoire « Corriger à la hausse » ) , le temps de revenir à des conditions nominales de remplissage (situation « Maintenir »).

La présente invention concerne alors une méthode de remplissage d'un lot de bouteilles de gaz avec un mélange gazeux identique, le mélange gazeux pouvant être constitué d'un constituant unique dans une matrice, ou bien de plusieurs constituants dans une matrice, matrice constituée d'un ou plusieurs gaz de fond, où l'on dispose d'une installation de remplissage comportant les éléments suivants :
- un ou des réservoirs stockant le ou les gaz de fond, appelés aussi gaz «matrice» ;
- une ou des bouteilles à haute pression stockant le ou lesdits constituants, également appelés « gaz de mélange » ;
- une ou des bouteilles à haute pression stockant un ou des gaz dits de référence, permettant d'étalonner un système analytique ;
- une chambre de mélange ;
- des lignes de gaz aptes à acheminer les gaz de fond et les gaz de mélange vers la chambre de mélange, lignes de gaz qui sont chacune munies d'un ou de plusieurs Régulateurs de Débit Massique (RDM) ;
- un étage de compression comprenant un compresseur de gaz ;
- une ligne de gaz apte à acheminer le mélange gazeux formé dans la chambre de mélange vers l'étage de compression, étage de compression qui est apte à pressuriser le mélange gazeux afin de pouvoir en remplir chacune des bouteilles dudit lot de bouteilles ;
- un système analytique comportant des analyseurs choisis ( apte à contrôler les Régulateurs de Débit Massique) ;
- une ligne de gaz apte à acheminer une partie du mélange gazeux formé dans la chambre de mélange vers le système analytique pour pouvoir faire l'analyse du mélange gazeux acheminé vers le système, ligne avantageusement mise en place en dérivation sur ladite ligne de gaz reliant la chambre de mélange et l'étage de compression ;
- une ou plusieurs rampes de remplissage permettant de positionner les bouteilles dudit lot de bouteilles à remplir et apte(s) à répartir uniformément le mélange gazeux dans chacune des bouteilles dudit lot de bouteilles ;
- une ligne de gaz apte à acheminer le mélange gazeux pressurisé de l'étage de compression vers la ou les rampe(s) de remplissage, la ou chaque rampe de remplissage pouvant être totalement ou partiellement occupée par les bouteilles dudit lot de bouteilles ;
- une ou des ligne(s) de gaz apte(s) à acheminer une partie du mélange gazeux pressurisé dans les bouteilles dudit lot de bouteilles vers le système analytique pour pouvoir faire une analyse de vérification post-remplissage des bouteilles après le cycle de remplissage (cette analyse permet également un contrôle de la composition du mélange en temps réel pendant le remplissage) ;
se caractérisant en ce que l'on procède à la mise en œuvre des mesures suivantes :
- on achemine le mélange gazeux en sortie de la chambre de mélange vers l'étage de compression, qui procède à une augmentation de la pression du mélange jusqu'à une consigne souhaitée ;
- on achemine le mélange gazeux en sortie de l'étage de compression vers la ou les rampes de remplissage et l'on procède à la répartition uniforme du mélange gazeux dans ledit lot de bouteilles, et l'on procède à l'arrêt du remplissage une fois une consigne de pression atteinte dans au moins une des bouteilles dudit lot de bouteilles ;
- on procède à l'analyse en temps réel, par le système analytique, de l'ensemble des composants du mélange gazeux en sortie de la chambre de mélange et avant son entrée dans l'étage de compression ;
- en fonction du résultat de ladite analyse en temps réel de la composition du mélange gazeux sortant de la chambre de mélange, à un instant donné, on procède à une estimation en temps réel de la composition du mélange gazeux dans les bouteilles dudit lot, par intégration depuis le début du remplissage de la composition en temps réel du mélange gazeux sortant de la chambre de mélange ou de la composition en temps réel du mélange gazeux entrant dans la ou les bouteilles dudit lot de bouteilles ; et
- on effectue une comparaison de cette estimation avec une ou des valeurs de consigne pour la ou les concentrations finales visées dans chacune des bouteilles du lot, et l'on procède, si nécessaire, en temps réel, à des rétroactions pour modifier les débits de gaz de chaque composant du mélange, gaz(s) de mélange et/ou gaz(s) de fond, parvenant à la chambre, par une modification du réglage des régulateurs de débit massique associés à chaque composant, de manière à maintenir et/ou corriger à la hausse et/ou corriger à la baisse les concentrations particulières des composants du mélange sortant de la chambre de mélange, et ainsi de poursuivre le remplissage avec une composition nominale ou enrichie ou appauvrie en l'un ou plusieurs des composants du mélange.

Comme il apparaîtra clairement à l'homme du métier, les rétroactions effectuées selon la présente invention permettent de faire en sorte que la composition estimée en temps réel converge en final au plus près vers la composition cible souhaitée.

Selon un des modes avantageux de mise en œuvre de l'invention, on prépare le mélange en procédant à l'évacuation du mélange gazeux qui sort de l'étage de compression dont la composition est éloignée de la consigne, vers un ou des évents. Une fois que la composition du mélange gazeux est stabilisée, en quelques minutes, typiquement environ 10 minutes, à la composition cible souhaitée, on commence le remplissage du dudit lot de bouteilles.

Ce mode avantageux propose donc une analyse que l'on peut qualifier d'analyse « additionnelle », à savoir une analyse qui va autoriser le démarrage du remplissage, analyse de ce qui sort de l'étage de compression, avant l'arrivée dans une rampe, où l'on compare avec une consigne et où l'on « jette » ce qui sort tant que le résultat n'est pas à moins d'une limite acceptée d'une consigne donnée.

Ce mode de réalisation peut se révéler tout particulièrement intéressant dans certaines situations et notamment pour des durées de remplissage courtes (petit lot en quantité de bouteilles, des pressions de consignes basses, des bouteilles de petites contenances...) où l'on dispose de moins de temps pour réagir et re-converger.

La [Figure 1] annexée illustre un exemple d'installation convenant pour la mise en œuvre de l'invention.

La nomenclature des éléments (en prenant pour cet exemple deux items par élément, mais le nombre d'items pour chaque élément peut être supérieur à deux) présents sur cette Figure 1 est la suivante :
- 101 : Bouteille de référence N°1
- 102 : Bouteille de référence N°2
- 103 : Gaz de fond N°1
- 104 : Gaz de fond N°2
- 105 : Bouteille gaz de mélange N°1
- 106 : Bouteille gaz de mélange N°2
- 107 : Régulateur de débit massique N°1
- 108 : Régulateur de débit massique N°2
- 109 : Chambre de mélange
- 110 : Analyseur N°1
- 111 : Analyseur N°2
- 112 : Compresseur
- 113 : Bouteille à remplir N°1
- 114 : Bouteille à remplir N°2
- 115 : Régulateur de débit massique du gaz de fond N°1
- 116 : Régulateur de débit massique du gaz de fond N°2
- 117 : Mesure optionnelle de débit massique du mélange gazeux entre l'étage de compression et le lot de bouteilles (placée sur la ligne L8)
- L1 : Ligne de gaz entre les bouteilles de références et les analyseurs
- L2 : Lignes de gaz entre les bouteilles de gaz de mélange et les régulateurs de débit massique contrôlant les gaz de mélange
- L3 : Ligne de gaz entre les bouteilles de gaz de fond et les analyseurs
- L4 : Lignes de gaz entre les bouteilles de gaz de fond et les régulateurs de débit massique contrôlant les gaz de fond
- L5 : Lignes de gaz entre les régulateurs de débit massique contrôlant les gaz de fond et la chambre de mélange
- L6 : Ligne de gaz entre la sortie de la chambre de mélange et l'entrée du compresseur
- L7 : Ligne de gaz entre la sortie de la chambre de mélange et les analyseurs
- L8 : Ligne de gaz entre la sortie du compresseur et le lot de bouteilles
- L9 : Ligne d'asservissement entre les analyseurs et les régulateurs de débit massique
- L10 : Lignes de gaz entre les régulateurs de débit massique contrôlant les gaz de mélange et la chambre de mélange

La [Figure 2] annexée illustre cette même installation représentée ici en mode « post-remplissage », dans une vue schématique partielle par rapport à la représentation donnée en Figure 1.

La nomenclature des références présentes sur la [Figure 2] est identique à celle présente sur la [Figure 1], avec par ailleurs la présence d'une Ligne L11 qui représente la ligne de gaz entre les bouteilles produites et les analyseurs de gaz.

## Revendications

1. Une méthode de remplissage d'un lot de bouteilles de gaz (113, 114...) avec un mélange gazeux, le mélange gazeux pouvant être constitué d'un constituant unique dans une matrice, ou bien de plusieurs constituants dans une matrice, matrice constituée d'un ou plusieurs gaz de fond, où l'on dispose d'une installation de remplissage comportant les éléments suivants :
- un ou des réservoirs stockant le ou les gaz de fond (103, 104..), appelés aussi gaz «matrice» ;
- une ou des bouteilles à haute pression stockant le ou lesdits constituants, également appelés « gaz de mélange » (105, 106...) ;
- une ou des bouteilles à haute pression stockant un ou des gaz dits de référence (101, 102..), permettant d'étalonner un système analytique ;
- une chambre de mélange (109) ;
- des lignes de gaz (L2, L4, L5, L10) aptes à acheminer les gaz de mélange et les gaz de fond vers la chambre de mélange, lignes de gaz qui sont chacune munies d'un ou de plusieurs Régulateurs de Débit Massique (107, 108, 115, 116) ;
- un étage de compression (112) comprenant un compresseur de gaz ;
- une ligne de gaz (L6) apte à acheminer le mélange gazeux formé dans la chambre de mélange vers l'étage de compression, étage de compression qui est apte à pressuriser le mélange gazeux afin de pouvoir en remplir chacune des bouteilles dudit lot de bouteilles ;
- ledit système analytique comportant des analyseurs choisis (110, 111...) ;
- une ligne de gaz (L7) apte à acheminer une partie du mélange gazeux formé dans la chambre de mélange vers ledit système analytique qui comporte des analyseurs choisis, pour pouvoir faire une analyse en temps réel de la composition du mélange gazeux ainsi prélevé, ligne avantageusement mise en place en dérivation sur ladite ligne de gaz (L6) reliant la chambre de mélange et l'étage de compression ;
- une ou plusieurs rampes de remplissage permettant de positionner les bouteilles dudit lot de bouteilles à remplir et apte(s) à répartir le mélange gazeux dans chacune des bouteilles (113, 114) dudit lot de bouteilles ;
- une ligne de gaz (L8) apte à acheminer le mélange gazeux pressurisé de l'étage de compression vers la ou les rampe(s) de remplissage, la ou les rampe(s) de remplissage pouvant être totalement pleine ou bien partiellement peuplée(s) par les bouteilles dudit lot de bouteilles ;
- une ou des ligne(s) de gaz (L11) apte(s) à acheminer une partie du mélange gazeux pressurisé dans les bouteilles dudit lot de bouteilles vers ledit système analytique qui comporte des analyseurs choisis, pour pouvoir notamment faire une analyse de vérification post-remplissage de la composition des bouteilles après le cycle de remplissage, ligne(s) de gaz apte(s) également à permettre la réalisation d'un contrôle de la composition gazeuse d'une bouteille en temps réel pendant le remplissage ;
**se caractérisant en ce que** l'on procède à la mise en œuvre des mesures suivantes :
- on achemine le mélange gazeux en sortie de la chambre de mélange vers l'étage de compression, qui procède à une augmentation de la pression du mélange jusqu'à une consigne souhaitée ;
- on achemine le mélange gazeux en sortie de l'étage de compression vers la ou les rampe(s) de remplissage et l'on procède à la répartition du mélange gazeux dans ledit lot de bouteilles, et l'on procède à l'arrêt du remplissage une fois une consigne de pression atteinte dans au moins une des bouteilles dudit lot ;
- on procède à l'analyse en ligne et en temps réel, par ledit système analytique qui comporte des analyseurs choisis, de l'ensemble des composants de mélange du mélange gazeux en sortie de la chambre de mélange (L7) et avant son entrée dans l'étage de compression ;
- en fonction du résultat de ladite analyse en temps réel de l'ensemble des composants de mélange du mélange gazeux en sortie de la chambre de mélange (L7), avant son entrée dans l'étage de compression, et ainsi de la composition du mélange gazeux issu de la chambre de mélange, à un instant donné, on procède à une estimation en temps réel de la composition du mélange gazeux dans une des bouteilles, par intégration depuis le début du remplissage de ladite composition en temps réel du mélange gazeux sortant de la chambre de mélange, ou par intégration de la composition du mélange gazeux entrant dans la ou les bouteilles du dudit lot de bouteilles, telle que déterminée durant ledit contrôle de la composition gazeuse d'une bouteille ou des bouteilles, en temps réel pendant le remplissage ; et
- on effectue une comparaison de cette estimation avec une ou des valeurs de consigne pour la ou les concentrations finales visées dans chacune des bouteilles du lot, et l'on procède, si nécessaire, automatiquement et en temps réel, à des rétroactions pour modifier les débits de gaz de chaque composant du mélange parvenant à la chambre, par une modification du réglage des régulateurs de débit massique associés à chaque composant, de manière à maintenir et/ou corriger à la hausse et/ou corriger à la baisse les concentrations particulières des composants du mélange sortant de la chambre de mélange, et ainsi permettre de poursuivre le remplissage avec une composition nominale ou enrichie ou appauvrie en l'un ou certains des composants du mélange.

2. Méthode selon la revendication 1, **se caractérisant en ce que** l'on effectue une opération préliminaire de préparation du mélange de la manière suivante :
- a) On procède à une analyse de la composition du mélange sortant de l'étage de compression avant que ce mélange n'arrive dans une ou les rampes ;
- b) On compare le résultat de cette analyse à une consigne et l'on élimine à l'évent le mélange tant que la différence entre le résultat de l'analyse et la consigne est supérieure à une valeur considérée comme acceptable ;
- c) On autorise le remplissage dudit lot de bouteilles quand le résultat de la comparaison de l'étape b) est inférieure à ladite valeur considérée comme acceptable.

## Patentansprüche

1. Ein Verfahren zum Füllen einer Charge von Gasflaschen (113, 114...) mit einem Gasgemisch, wobei das Gasgemisch aus einem einzigen Bestandteil in einem Träger oder aber aus mehreren Bestandteilen in einem Träger bestehen kann, wobei der Träger aus einem oder mehreren Hintergrundgasen besteht, wobei eine Füllanlage bereitgestellt ist, die die folgenden Element umfasst:
- einen oder mehrere Behälter, die das oder die Hintergrundgase (103, 104..), auch als "Träger"-Gase bezeichnet, speichern;
- eine oder mehrere Hochdruckflaschen, die den oder die Bestandteile, auch als "Gemischgase" (105, 106...) bezeichnet, speichern;
- eine oder mehrere Hochdruckflaschen, die ein oder mehrere sogenannte Referenzgase (101, 102..) speichern, die das Kalibrieren eines analytischen Systems ermöglichen;
- eine Mischkammer (109);
- Gasleitungen (L2, L4, L5, L10), die dazu fähig sind, die Gemischgase und die Hintergrundgase zu der Mischkammer zu leiten, wobei die Gasleitungen jeweils über einen oder mehrere Massendurchflussregler (107, 108, 115, 116) verfügen;
- eine Verdichtungsstufe (112), die einen Gasverdichter beinhaltet;
- eine Gasleitung (L6), die dazu fähig ist, das in der Mischkammer gebildete Gasgemisch zu der Verdichtungsstufe zu leiten, wobei die Verdichtungsstufe dazu fähig ist, das Gasgemisch unter Druck zu setzen, um jede der Flaschen der Flaschencharge damit füllen zu können;
- wobei das analytische System ausgewählte Analysegeräte (110, 111...) umfasst;
- eine Gasleitung (L7), die dazu fähig ist, einen Teil des in der Mischkammer gebildeten Gasgemisches zu dem analytischen System, das ausgewählte Analysegeräte umfasst, zu leiten, um eine Echtzeitanalyse der Zusammensetzung des so entnommenen Gasgemisches vornehmen zu können, wobei die Leitung vorteilhafterweise als Abzweig an der Gasleitung (L6), die die Mischkammer und die Verdichtungsstufe verbindet, eingerichtet ist;
- eine oder mehrere Füllleisten, die es ermöglichen, die Flaschen der zu füllenden Flaschencharge zu positionieren, und dazu fähig sind, das Gasgemisch in jede der Flaschen (113, 114) der Flaschencharge zu verteilen;
- eine Gasleitung (L8), die dazu fähig ist, das unter Druck gesetzte Gasgemisch von der Verdichtungsstufe zu der oder den Füllleisten zu leiten, wobei die eine oder die mehreren Füllleisten entweder komplett voll oder aber teilweise durch die Flaschen der Flaschencharge belegt sein können;
- eine oder mehrere Gasleitungen (L11), die dazu fähig sind, einen Teil des unter Druck gesetzten Gasgemisches in den Flaschen der Flaschencharge zu dem analytischen System, das ausgewählte Analysegeräte umfasst, zu leiten, um insbesondere eine dem Füllen nachgeschaltete Analyse zur Überprüfung der Zusammensetzung der Flaschen nach dem Füllzyklus vornehmen zu können, wobei die eine oder die mehreren Gasleitungen ferner dazu fähig sind, die Durchführung einer Echtzeitkontrolle der Gaszusammensetzung einer Flasche während des Füllens zu ermöglichen;
**dadurch gekennzeichnet, dass** die folgenden Maßnahmen umgesetzt werden:
- Leiten des Gasgemisches am Ausgang der Mischkammer zu der Verdichtungsstufe, die eine Erhöhung des Drucks des Gemisches bis zu einem gewünschten Sollwert vornimmt;
- Leiten des Gasgemisches am Ausgang der Verdichtungsstufe zu der oder den Füllleisten und Vornehmen der Verteilung des Gasgemisches in die Flaschencharge und Vornehmen eines Füllstopps, wenn in mindestens einer der Flaschen der Charge ein Drucksollwert erreicht ist;
- Vornehmen der Online- und Echtzeitanalyse, durch das analytische System, das ausgewählte Analysegeräte umfasst, aller Gemischkomponenten des Gasgemisches am Ausgang der Mischkammer (L7) und vor seinem Eintritt in die Verdichtungsstufe;
- in Abhängigkeit von dem Ergebnis der Echtzeitanalyse aller Gemischkomponenten des Gasgemisches am Ausgang der Mischkammer (L7), vor seinem Eintritt in die Verdichtungsstufe, und somit der Zusammensetzung des Gasgemisches, das aus der Mischkammer kommt, zu einem gegebenen Zeitpunkt, Vornehmen einer Echtzeitschätzung der Zusammensetzung des Gasgemisches in einer der Flaschen durch Integration, ab dem Beginn des Füllens, der Echtzeitzusammensetzung des Gasgemisches, das aus der Mischkammer austritt, oder durch Integration der Zusammensetzung des Gasgemisches, das in die eine oder die mehreren Flaschen der Flaschencharge eintritt, wie während der Kontrolle der Gaszusammensetzung einer oder mehrerer Flaschen während des Füllens in Echtzeit bestimmt; und
- Ausführen eines Vergleichs dieser Schätzung mit einem oder mehreren Sollwerten für die eine oder die mehreren angestrebten Endkonzentrationen in jeder der Flaschen der Charge und, falls erforderlich, Vornehmen, automatisch und in Echtzeit, von Rückkopplungen, um die Gasdurchflüsse jeder Komponente des Gemisches, die zu der Kammer gelangt, durch eine Modifizierung der Einstellung der Massendurchflussregler, die mit jeder Komponente assoziiert sind, zu modifizieren, um die bestimmten Konzentrationen der Komponenten des Gemisches, das aus der Mischkammer austritt, beizubehalten und/oder nach oben zu korrigieren und/oder nach unten zu korrigieren und so das Fortsetzen des Füllens mit einer nominellen oder mit einer oder bestimmten der Komponenten des Gemisches angereicherten oder abgereicherten Zusammensetzung zu ermöglichen.

2. Verfahren nach Anspruch 1, **dadurch gekennzeichnet, dass** ein Vorabvorgang zur Vorbereitung des Gemisches wie folgt ausgeführt wird:
- a) Vornehmen einer Analyse der Zusammensetzung des Gemisches, das aus der Verdichtungsstufe austritt, bevor diese Mischung in einer oder den Füllleisten ankommt;
- b) Vergleichen des Ergebnisses dieser Analyse mit einem Sollwert und Abführen des Gemisches zur Entlüftung, solange die Differenz zwischen dem Ergebnis der Analyse und dem Sollwert größer als ein als akzeptabel betrachteter Wert ist;
- c) Gestatten des Füllens der Flaschencharge, wenn das Ergebnis des Vergleichs aus Schritt b) kleiner als der als akzeptabel betrachtete Wert ist.

## Claims

1. Method for filling a batch of gas bottles (113, 114...) with a gas mixture, wherein the gas mixture may consist of a single constituent in a matrix or a plurality of constituents in a matrix, which matrix consists of one or more base gases, in which a filling installation having the following elements is provided:
- one or more reservoirs storing the base gas or gases (103, 104...), also referred to as "matrix" gases;
- one or more high-pressure bottles storing said constituent or constituents, also referred to as "mixing gases" (105, 106...);
- one or more high-pressure bottles storing one or more so-called reference gases (101, 102...), making it possible to calibrate an analytical system;
- a mixing chamber (109);
- gas lines (L2, L4, L5, L10) suitable for conveying the mixing gases and the base gases to the mixing chamber, which gas lines are each equipped with one or more mass flow controllers (107, 108, 115, 116);
- a compression stage (112) comprising a gas compressor;
- a gas line (L6) suitable for conveying the gas mixture formed in the mixing chamber to the compression stage, which compression stage is suitable for pressurizing the gas mixture in order to be able to fill each of the bottles of said batch of bottles therewith;
- said analytical system having selected analysers (110, 111...);
- a gas line (L7) suitable for conveying a part of the gas mixture formed in the mixing chamber to said analytical system which comprises selected analysers, in order to be able to carry out real-time analysis of the composition of the gas mixture sampled in this way, which line is advantageously placed in bypass on said gas line (L6) connecting the mixing chamber to the compression stage;
- one or more filling stations making it possible to position the bottles of said batch of bottles to be filled and suitable for distributing the gas mixture into each of the bottles (113, 114) of said batch of bottles;
- a gas line (L8) suitable for conveying the pressurized gas mixture from the compression stage to the filling station or stations, the filling station or stations being capable of being entirely full or partially populated with the bottles of said batch of bottles;
- one or more gas lines (L11) suitable for conveying a part of the pressurized gas mixture in the bottles of said batch of bottles to said analytical system which comprises selected analysers, in order to be able in particular to perform a post-filling verification analysis of the composition of the bottles after the filling cycle, which one or more gas lines are also suitable for allowing monitoring of the gaseous composition of a bottle in real time during the filling;
**characterized in that** the following measures are carried out:
- the gas mixture at the exit of the mixing chamber is conveyed to the compression stage, which carries out an increase of the pressure of the mixture to a desired setpoint;
- the gas mixture at the exit of the compression stage is conveyed to the filling station or stations and the distribution of the gas mixture into said batch of bottles is carried out, and the filling is stopped once a pressure setpoint has been reached in at least one of the bottles of said batch;
- online real-time analysis of all the mixing components of the gas mixture is carried out by said analytical system, which comprises selected analysers, at the exit of the mixing chamber (L7) and before entry into the compression stage;
- depending on the result of said real-time analysis of all the mixing components of the gas mixture leaving the mixing chamber (L7), before it enters the compression stage, and thus on the composition of the gas mixture leaving the mixing chamber, at a given time, a real-time estimation of the composition of the gas mixture in one of the bottles is carried out by integrating the gas mixture leaving the mixing chamber from the beginning of the filling of said composition, in real time, of the gas mixture leaving the mixing chamber, or by integrating the composition of the gas mixture entering the bottle(s) of said batch of bottles, as determined during said monitoring of the gas composition of a bottle or bottles, in real time during filling; and
- a comparison of this estimation with one or more setpoint values for the final concentration or concentrations intended in each of the bottles of the batch is carried out, and, if necessary, feedbacks are carried out automatically and in real time in order to modify the gas flow rates of each component of the mixture reaching the chamber, by modifying the setting of the mass flow controllers associated with each component, so as to maintain and/or correct upwards and/or correct downwards the particular concentrations of the components of the mixture leaving the mixing chamber, and thus to be able to continue the filling with a nominal composition or a composition enriched or depleted with respect to one or some of the components of the mixture.

2. Method according to Claim 1, **characterized in that** a preliminary operation of preparing the mixture is carried out in the following way:
- a) an analysis of the composition of the mixture leaving the compression stage is carried out before this mixture arrives in one or more of the stations;
- b) the result of this analysis is compared with a setpoint and the mixture is vented if the difference between the result of the analysis and the setpoint is greater than a value considered to be acceptable;
- c) filling of said batch of bottles is authorized when the result of the comparison of step b) is less than said value considered to be acceptable.
